# EUROPEAN PATENT APPLICATION

(11) **EP 0 805 214 A2**
(43) Date of publication of application: **05.11.1997**
(21) Application number: 97107094.1
(22) Date of filing: 29.04.1997
(51) Int. Cl.: C12Q 1/68

(54) **Method and relative primers to identify listeria monocytogenes in an organic substrate**

(30) Priority: 30.04.1996 IT US960064
(71) Applicant: Universita' Degli Studi di Udine, 33100 Udine (IT)
(72) Inventor: Manzano, Marisa, 33100 Udine (IT); Oomi, Giuseppe, 20063 Cernusco Sul Naviglio (Mi) (IT)
(74) Representative: Petraz, Gilberto Luigi

(57) **Abstract**

Primer to identify *Listeria monocytogenes* either naturally present or artificially inoculated in organic substrates such as food, blood, urine and exudates in general, the primers being composed respectively of 5'-GGGCTTTATCCATAAAATA-3' (xyz, sense) and of 5'-TTGGAAGAACCTTGATTA-3' (wts, antisense), the sequence indicated being obligatory at least in the respective last four bases towards 3' of the two primers.
Method to identify *Listeria monocytogenes*, either naturally present or artificially inoculated in organic substrates such as food, blood, urine and exudates in general, in which the sample is diluted and homogenised, a part is taken of the sample of the organic substrate homogenised in an enriched broth or sterile water, the homogenised broth receives an addition of proteinase and is incubated at a temperature of between about 60°C and 70°C for about an hour, then is boiled for about 10 minutes, centrifugated and then subjected to a PCR reaction with the introduction of the primers according to above and other reaction substances, of which at least one is magnesium chloride, a part of the resulting product being subjected to electrophoresis and visualised with a transilluminator to supply the identifying signals.

## Description

This invention concerns a method to identify *Listeria monocytogenes* in organic substrates, and also the specific primers to identify *Listeria monocytogenes* as set forth in the respective main claims.

The invention is applied in the field of bacteriological tests and makes it possible to develop an extremely rapid, simple and precise method to identify the presence of *Listeria monocyogenes* in organic substrates such as food, blood, urine and exudates in general.

*Listeria* is an ubiquitous microorganism which can be isolated from a wide range of foodstuffs such as raw or cured meat, fresh milk, cheese, vegetables, and also from the soil, from animal tissues, from silage, from waste water.

One characteristic of this microorganism is that it can multiply even at the temperatures of refrigeration normally used in the preservation of food.

In recent years, and in various parts of the world, there have been epidemics of listeriosis, caused by the consumption of food contaminated by *Listeria monocytogenes*. These epidemics have caused serious and often fatal infections in people considered at risk, such as new-born babies, pregnant women, and immunodepressed subjects.

Meat and some meat products, milk and some milk products, and vegetables are the most common vehicles of transmission of *Listeria*.

Contamination can occur during harvesting, slaughter, production and sale.

However, cases of Listeriosis are rare, and the potential risk for consumers is low, both because proper cooking and pasteurisation techniques may completely inhibit *Listeria* in foodstuffs, and also because the infection dose is about 1,000-10,000 *Listeria monocytogenes* per gram.

Nevertheless, food and food processing machinery are monitored all over the world, according to existing laws and precise regulations, for the presence of *Listeria monocytogenes*.

Traditional methods of culture are too slow, however, and results are obtained only after 4 or 5 working days using enrichment broths, selective agars and morphological and biochemical tests. Sometimes, traditional identification methods can give false-positive or false-negative results, especially if the number of competing microorganisms is high.

A further problem is that uncertain results may be obtained with the Camp-test, and therefore *Listeria monocytogenes* cannot be distinguished from *Listeria innocua*; this explains the need for new methods, which are rapid, accurate and discriminating so as to identify *Listeria monocytogenes* in foodstuffs.

New methods have recently been introduced which are based on molecular biology for the diagnosis of *Listeria*; these non-traditional methods comprise hybridisation with specific DNA probes, analysis of DNA fragments obtained after digestion with restriction enzymes (endonucleasis), pulsed-field electrophoresis or the polymerase chain reaction (PCR).

Even though the majority of these techniques are not very suitable for routine use in laboratories and can be time-consuming, PCR offers a simple, rapid and easy-to-perform procedure, with high sensitivity and specificity.

The technique has been used to identify and distinguish species and strains of the *Listeria* genus using both single primers of arbitrary sequence (RAPD) and specific primers for listeriolysine O and for iap genes.

The iap gene, which encodes for the invasion-associated protein (p60), occurs in all *Listeria* species and is characterised by conserved and variable regions.

The use of specific olionucleotides is advantageous if the DNA sequence is known, whereas RAPD typing is more universally applicable since no DNA sequence information is needed.

The present applicant has designed, tested and embodied this invention in order to achieve a method to identify *Listeria monocytogenes* which is more rapid and more simple than methods known to the state of the art.

This invention is set forth and characterised in the main claim, while the dependent claims describe variants of the idea of the main embodiment.

The purpose of the invention is to provide a method and the relative primers to identify univocally *Listeria monocytogenes* in organic substrates using the procedure of polymerase chain reaction (hereinafter PCR).

The invention uses a PCR method using a new set of primers to detect and identify, in a shorter time and with a greater likelihood of success, *Listeria monocytogenes* in organic substrates in general, such as food, blood, urine.

The advantages of this method are that, with the relative primers, it is highly specific and selective for *Listeria monocytogenes* and is not influenced either by the type of substrate or by the presence of other species of *Listeria* or other microorganisms.

Moreover, the method is rapid, simple to use and sure.

The specificity of the primers, here called MAR1-MAR2, which are derived from the iap gene, for *Listeria moncytogenes* has been evaluated by using pure DNA extracted from a wide range of bacteria.

In order to develop the PCR method, primers made from conserved sequences of the iap gene were used.

The primers were synthetised with t-methoxyphosphoramidite chemistry using an Applied Biosystem (USA) 392-A DNA synthetizer.

The composition which was identified as optimal for the base of the primers, denoted for the purposes of the invention as Mar1 (sense) and Mar2 (antisense), is respectively 5'-GGGCTTTATCCATAAAATA-3' (xyz) and 5'-TTGGAAGAACCTTGATTA-3' (wts).

To be more exact, for the purposes of the invention, the sequence which defines the last four bases of the individual primers is obligatory, that is to say, the four bases towards the 3'.

For the other bases of the individual primers, thirteen in this specific case, but other numbers are possible, variations from one to four non consecutive bases, according to the invention, are acceptable.

Moreover, according to the invention, a variation in the sequence of the bases which are not the last four causes a correlated variation in the annealing temperature of the primers.

The MAR1 and MAR2 primers, localised within the iap gene of *Listeria moncytogenes* at the positions from 635 to 651 bp and from 1071 to 1087 bp, respectively, cause the amplification of a 453 bp fragment.

To obtain the specific signals relating to the results following the application of the method and the primers according to the invention, simplified experiments were carried out with pure strains of *Listeria monocytogenes* of clinical or environmental origin and with an organic substrate artificially inoculated with known quantities of viable *Listeria monocytogenes* cells.

Moreover, the same experiments were carried out using strains of other species of *Listeria* and of other microorganisms.

To extract the DNA from the pure strain, the bacteria cultures were first diluted with sterile water to a concentration of 10⁷ cells per millilitre; this value was verified by reading the preparation under the spectrophotometer.

In the example shown, the number of cells corresponding to an absorbance of A₆₀₀ₙₘ = 0.2 was determined by counting them under a microscope by means of a hematocytometer.

To perform the PCR analysis, in this case, 10 µl of a proteinase K (Boehringer, Germany) solution, with a concentration of 10 mg per millilitre, were added to 200 µl of the cell suspension; the solution was then incubated to a temperature of between 60 and 70°C, advantageously about 63°C, for about 1 hour.

After this time, the suspension was boiled for 10 minutes and then kept at 4°C.

Five µl of the solution were used for the PCR assay.

The PCR assay was made in a volume of 50 µl containing the following reaction substances:
- 100mM Tris-HCl pH 8.3, 500mM KCl;
- MgCl₂ to a concentration of between 1.0 and 4.0 mM, advantageously 1.5 mM;
- 1 µl of DNA sample;
- 1.25µM of each deoxynucleoside triphosphate dATP, dGTP, dCTP and dTTP;
- primer at a concentration of between 0.5 and 2.0 pM and 2.5 U of Taq-DNA polymerase.

The solution was overlaid with 50 µl of sterile mineral oil and subjected to a Thermalcycler process.

The DNA sample was initially denatured at 95°C for about 5 minutes, then was subjected to about 35 cycles each consisting of about 90 seconds of denaturation at 95°C, 80 seconds primer annealing at a temperature of between 30° and 52°C, advantageously about 46°C, and a 2 minute extension at about 72°C.

At the end of the amplification the mixture was subjected to an additional 7 minute extension at 72°C.

20 µl of amplified PCR product were analysed by electrophoresis using a 2% agarose gel containing 1 µg/ml of ethidium bromide and using Marker VI as a molecular-weight marker for the DNA.

The amplified sequences were examined under UV light using a transilluminator apparatus and photographed with Kodak Royal X-Omat film.

The results of the PCR assay were compared with negative and positive control results: the positive control was an "in house" standard containing approximately 10⁵ *Listeria monocytogenes* cells per ml.

In the negative control, the bacterial DNA was replaced with 5 µl of sterile distilled water.

The *Listeria monocytogenes* is identified by the presence of a single, intense signal of molecular weight at 453 bp as should be expected from the primer position.

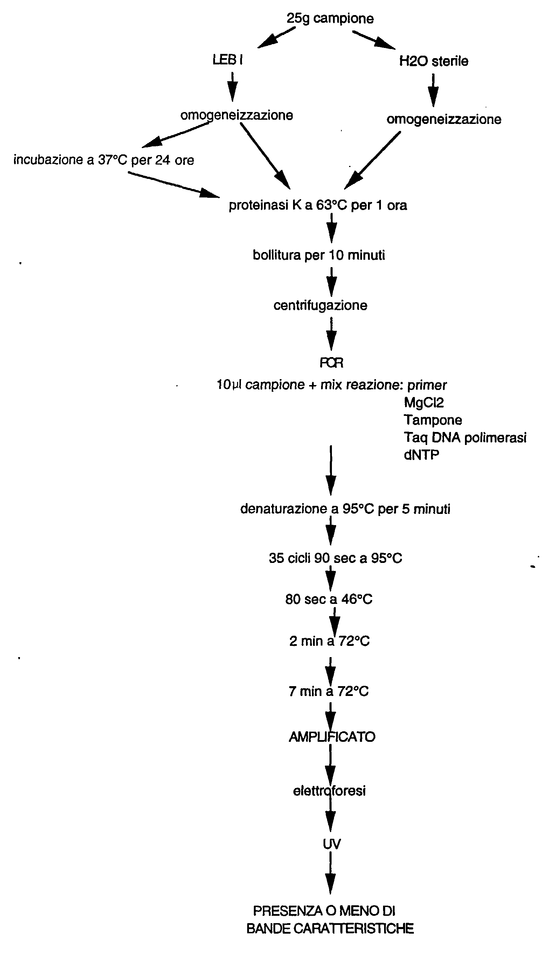

Vice versa, the absence of a single 453 bp molecular weight fragment, or the presence of more than one aspecific fragments, denotes the presence of other strains of *Listeria* or of other microorganisms, as shown in Fig. 1, which follows.

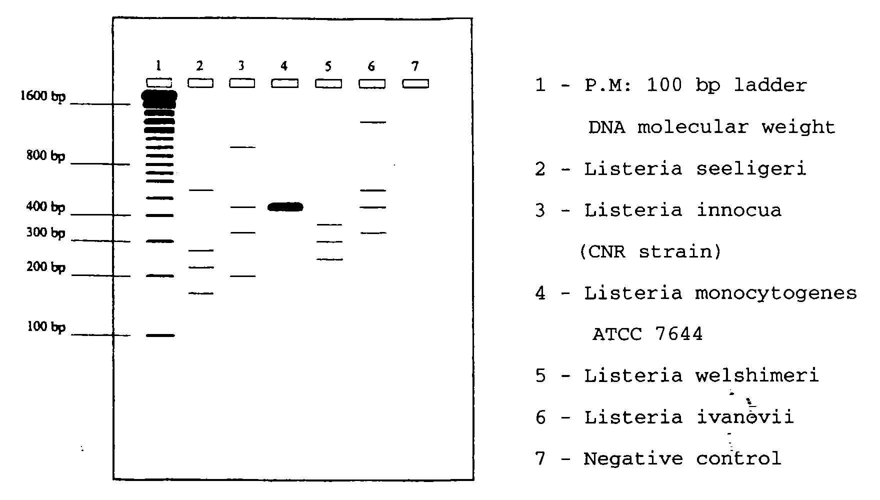
1 2 3 4 5 6 7
1600 bp
800 bp
400 bp
300 bp
200 bp
100 bp
1 - P.M: 100 bp ladder
DNA molecular weight
2 - Listeria seeligeri
3 - Listeria innocua
(CNR strain)
4 - Listeria monocytogenes
ATCC 7644
5 - Listeria welshimeri
6 - Listeria ivanovii
7 - Negative control

When applying the invention to samples of organic substrate containing *Listeria monocytogenes* the results of the signal are those shown in Fig. 1.

To apply the invention to samples of organic substrate in order to verify the presence or not of *Listeria monocytogenes* the following method was followed.

In order to prepare the sample to be subjected to the PCR assay, a direct method was used and also a method which included an enrichment stage.

In the basic operation, 25 g of organic substrate was taken, and to it were added 225 ml of LEB I (Listeria Enrichment Broth), or an equal quantity of sterile water, and then the solution was homogenised in a stomacher to obtain a homogenised broth.
- Direct method: 10 µl of homogenised broth were treated directly for PCR assay.
- Enrichment method: the homogenised broth was incubated at 37°C for 24 hours and 10 µl of this resultant broth were treated for PCR assay.

The best concentration of MgCl₂ and of primers is respectively less than or equal to 2.0 mM and less than or equal to 1pM.

The attached Table, given as an example, shows the flow chart which describes the method used in the invention. The primers for the iap gene and the method adopted have made it possible to distinguish *Listeria monocytogenes* from the other species of *Listeria* and also from other microorganisms.

By using primers for the iap gene, the detection limit of pure *Listeria monocytogenes* (ATCC 7644) culture was 1-10 cells/PCR reaction.; this limit is in fact almost equivalent to a pure culture of 10² *Listeria monocytogenes* in 1 ml of distilled water.

As shown in lines 6 and 7 of Fig. 2 which follows, 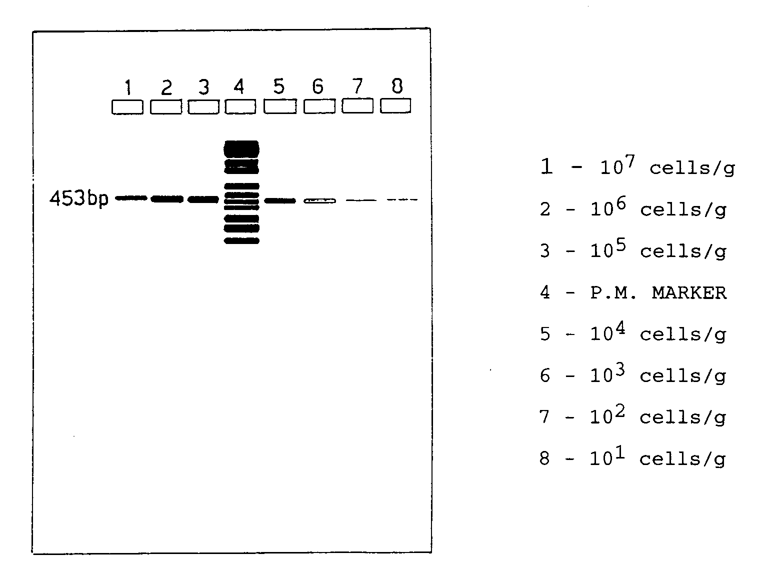
1 2 3 4 5 6 7 8
453bp
1 - 10^7 cells/g
2 - 10^6 cells/g
3 - 10^5 cells/g
4 - P.M. MARKER
5 - 10^4 cells/g
6 - 10^3 cells/g
7 - 10^2 cells/g
8 - 10^1 cells/g
 with concentrations of *Listeria monocytogenes* of about 10³ cells g⁻¹, a weak signal is obtained which tends to fade even more when the concentration of *Listeria monocytogenes* is about 10² cells g⁻¹.

Vice versa,when the concentration of *Listeria* is more than 10³ cells per gram, the signal is decidedly strong.

Therefore, it is possible to hypothesise that when the concentration of *Listeria monocytogenes* in food is less than 100 cells g⁻¹ there is a possibility of obtaining false negative results.

However, by using the indirect method which includes a 24 hour enrichment (it should be noted that the enrichment method only enriches viable cells, not dead ones) it is possible to obtain satisfactory results by using the PCR method and the traditional method in parallel.

In a comparison between the two methods no false negative or false positive results were observed.

Moreover, experiments made on meat samples of various origin showed that the type of product has no influence on the PCR results.

Even when there is an excess of competing DNA due to a large number of competing bacteria, this does not have an inhibitory effect.

It should be noted that traditional methods, apart from being excessively time-consuming, may give ambiguous results, especially in distinguishing *Listeria monocytogenes* from *Listeria innocua*.

In fact, the Camp-test and the β-haemolysis test sometimes fail when they are used to identify *Listeria monocytogenes*.

The routine PCR technique according to the invention is very simple and quick to use; it takes only 30 hours, compared with the 4-5 days required by conventional methods, to detect and distinguish *Listeria monocytogenes* in meat, since it is useless to isolate and culture *Listeria* under glass.

## Claims

1. Primer to identify *Listeria monoctogenes* either naturally present or artificially inoculated in organic substrates such as food, blood, urine and exudates in general, the primer being characterised in that it is composed respectively of 5'-GGGCTTTATCCATAAAATA-3' (xyz, sense) and of 5'-TTGGAAGAACCTTGATTA-3' (wts, antisense), the indicated sequence being obligatory at least in the respective last four bases towards 3' of the two primers.

2. Primer as in Claim 1, in which from one to four non-consecutive bases facing towards 5' and different from the last four towards 3' may be varied with respect to the sequence indicated in Claim 1.

3. Primer as in Claim 1 or 2, in which the annealing temperature of the primer is correlated to the sequence of the bases of the individual primers which are different from the last four.

4. Method to identify *Listeria monocytogenes*, either naturally present or artificially inoculated in organic substrates such as food, blood, urine and exudates in general, in which the sample is diluted and homogenised, the method being characterised in that a part is taken of the sample of organic substrate homogenised in an enrichment broth or in sterile water, proteinase is added to the homogenised broth which is then incubated at a temperature of between 60°C and 70°C for about an hour, is then boiled for about 10 minutes, centrifugated and then subjected to a PCR reaction with the introduction of the primers according to any one of Claims 1 to 3 inclusive and other reaction substances, amongst which is at least magnesium chloride, a part of the resulting product being subjected to electrophoresis and visualised with a transilluminator so as to supply the identification signals.

5. Method as in Claim 4, in which the concentration of the primers is between about 0.5 pM and about 2.0 pM.

6. Method as in Claim 4 or 5, in which the annealing temperature of the primers is between about 30°C and about 52°C.

7. Method as in any claim from 4 to 6 inclusive, in which the concentration of MgCl₂, added as a reaction substance during the PCR reaction step, is between about 1.0 mM and about 4.0 mM.

8. Method as in any claim from 4 to 7 inclusive, in which the identifying signals are those shown in Fig. 1.
